# EUROPÄISCHE PATENTSCHRIFT

(11) **EP 2 773 271 B1**
(45) Veröffentlichungstag und Bekanntmachung des Hinweises auf die Patenterteilung: **23.11.2016**
(21) Anmeldenummer: 12788151.4
(22) Anmeldetag: 30.10.2012
(51) Int. Cl.: A61B 17/28, A61B 17/29

(54) **HANDHABE FÜR EIN MEDIZINISCHES INSTRUMENT**
HANDLE FOR A MEDICAL INSTRUMENT
POIGNÉE POUR UN INSTRUMENT MÉDICAL

(30) Priorität: 31.10.2011 DE 102011085512
(43) Veröffentlichungstag der Anmeldung: 10.09.2014
(73) Patentinhaber: Richard Wolf GmbH, 75438 Knittlingen (DE)
(72) Erfinder: PRESTEL, Stephan, 76287 Rheinstetten (DE); FALK, Ernst, 75447 Sternenfels-Diefenbach (DE); KÖRNER, Eberhard, 75438 Knittlingen (DE); BOEBEL, Manfred, 75245 Neulingen (DE)
(74) Vertreter: Patentanwälte Vollmann & Hemmer
(86) Internationale Anmeldenummer: PCT/EP2012/071462
(87) Internationale Veröffentlichungsnummer: WO 2013/064487

(56) Entgegenhaltungen:
- EP-A1- 2 103 264
- EP-A1- 2 316 358
- EP-A2- 1 980 213
- DE-U1-202007 007 327

## Beschreibung

Die Erfindung betrifft eine Handhabe für ein medizinisches Instrument.

Unter einer Handhabe eines medizinischen Instruments versteht man üblicherweise den Teil des Instruments, an dem das Instrument mittels eines dort angeordneten Griffteils manuell gehalten und geführt wird und an dem ein oder mehrere Betätigungselemente zum Ausführen der mit dem Instrument ausführbaren Funktionen angeordnet sind.

Neben Handhaben, mit deren Betätigungselementen lediglich jeweils eine Funktion eines medizinischen Instruments ausführbar ist, ist aus DE 10 2009 051 515 A1 ein medizinisches Instrument mit einer Handhabe bekannt, bei dem mit einem Betätigungselement der Handhabe mehrere Funktionen ausgeführt werden können. Dieses Instrument weist einen proximalseitig der Handhabe angeordneten Schaft auf, der um seine Längsachse drehbar ist. An dem distalen Ende des Schafts ist ein Werkzeug angeordnet, das relativ zu dem Schaft verdrehbar ist. Die Handhabe weist ein Betätigungselement in Form eines Drehrads auf, mit dem einerseits der Schaft verdreht werden kann und andererseits das Werkzeug relativ zu dem Schaft verdreht werden kann. Hierzu ist das Drehrad in der Handhabe in zwei Funktionsstellungen axial verschiebbar, wobei es in einer ersten Funktionsstellung mit dem Schaft bewegungsgekoppelt ist, der dann durch ein Verdrehen des Drehrads verdreht werden kann, und in einer zweiten Funktionsstellung mit dem Werkzeug bewegungsgekoppelt ist, das in dieser Funktionsstellung durch Verdrehen des Drehrads relativ zu dem Schaft verdreht werden kann. Gegenüber Handhaben, mit deren Betätigungselementen lediglich jeweils eine Funktion ausgeführt werden kann, stellt die Handhabe des aus DE 10 2009 051 515 A1 bekannten Instruments eine deutliche Verbesserung dar, wobei diese Handhabe aber unter ergonomischen Gesichtspunkten auch noch nicht die bestmögliche Lösung darstellt. DE 10 2009 051 515 A1 offenbart die im Oberbegriff des Anspruchs 1 angegebenen Merkmale.

Aus DE 20 2007 007 327 U1 ist ein medizinisches Instrument bekannt, bei dem eine Handhabe ein Betätigungselement aufweist, mit dem zwei unterschiedliche Funktionen ausgeführt werden können, nämlich zwei unterschiedliche Werkzeuge des Instruments betätigt werden können. Hierzu ist das Instrument mit zwei Betätigungselementen ausgestattet, die wahlweise über einen Mitnehmer aktiviert werden können. Der Mitnehmer ist in einer Führung angeordnet, die über einen an der Handhabe angeordneten Betätigungshebel in zwei Stellungen verschwenkt werden kann. In diesen Stellungen ist der Mitnehmer jeweils nur mit einem der Betätigungselemente bewegungsgekoppelt. Der Betätigungshebel ist erforderlich, da die Führung mit dem Mitnehmer unzugänglich innerhalb der Handhabe angeordnet ist. Die Aktivierung des betreffenden Betätigungselementes erfolgt dann durch Verschieben des Mitnehmers in der Führung mittels eines schwenkbaren Griffteils der Handhabe.

Die Aufgabe der Erfindung ist es, eine Handhabe für ein medizinisches Instrument zu schaffen, die zumindest ein Betätigungselement zum Ausführen mehrerer Funktionen aufweist, wobei dieser Handhabe gegenüber der bekannten Handhabe dieser Art eine weiter verbesserte Bedienbarkeit aufweisen soll.

Gelöst wird diese Aufgabe durch eine Handhabe für ein medizinisches Instrument mit den in Anspruch 1 angegebenen Merkmalen. Vorteilhafte Weiterbildungen dieser Handhabe ergeben sich aus den Unteransprüchen, der nachfolgenden Beschreibung sowie den Zeichnungen. Hierbei können gemäß der Erfindung die in den Unteransprüchen und der Beschreibung angegebenen Merkmale jeweils für sich aber auch in Kombination die erfindungsgemäße Lösung gemäß Anspruch 1 weiter ausgestalten.

Die erfindungsgemäße Handhabe für ein medizinisches Instrument weist ein an der Handhabe angeordnetes Betätigungselement auf, das zumindest in eine erste und eine zweite Funktionsstellung verschiebbar ist und sowohl in der ersten als auch in der zweiten Funktionsstellung zur Ausübung einer Funktion in einer Ebene quer zu seiner Verschieberichtung bewegbar ist. Hierbei unterscheiden sich die in der ersten und zweiten Funktionsstellung mit dem Betätigungselement ausführbaren Funktionen. Die Art dieser in den zumindest zwei Funktionsstellungen mit dem Betätigungselement ausführbaren Funktionen richtet sich typischerweise nach der Art des medizinischen Instruments, bei dem die erfindungsgemäße Handhabe eingesetzt wird und ist grundsätzlich beliebig. Beispielsweise kann es sich bei den mit dem Betätigungselement ausführbaren Funktionen wie bei dem aus DE 10 2009 051 515 A1 bekannten Instrument um das Verdrehen eines distalseitig der Handhabe angeordneten Schafts in einer ersten Funktionsstellung und um das Verdrehen eines an dem distalen Ende des Schafts angeordneten Werkzeugs in der zweiten Funktionsstellung handeln. Des Weiteren ist der Einsatz der erfindungsgemäßen Handhabe auch bei einem solchen medizinischen Instrument denkbar, bei dem ein distalseitig der Handhabe angeordneter Schaft in einer ersten Funktionsstellung drehbar ist und ein distaler Endabschnitt dieses Schafts in einer zweiten Funktionsstellung gegenüber dem übrigen Schaft abwinkelbar ist. Bevorzugt ist das Betätigungselement neben den beiden Funktionsstellungen auch in eine solche Stellung verschiebbar, in der mit ihm keine Funktion ausführbar ist. Ferner ist eine Ausgestaltung der Handhabe denkbar, bei der das Betätigungselement in mehr als zwei Funktionsstellungen verschiebbar ist.

Die Grundidee der Erfindung ist es, die Bedienbarkeit der Handhabe dadurch zu verbessern, dass an der Handhabe ein schwenkbarer Betätigungshebel angeordnet ist, der zum Verschieben des Betätigungselements in die zumindest zwei Funktionsstellungen mit dem Betätigungselement bewegungsgekoppelt ist. Der Betätigungshebel ist zweckmäßigerweise als ein zweiseitiger Hebel ausgebildet, wobei ein erster Hebelarm des Betätigungshebels an dem Betätigungselement angreift und ein zweiter Hebelarm, der zweckmäßigerweise länger als der erste Hebelarm ist, zur manuellen Kraftübertragung auf den Betätigungshebel dient. Die Bewegungskopplung des Betätigungshebels mit dem Betätigungselement ist vorzugsweise derart, dass das Betätigungselement beim Verschwenken des Betätigungshebels in eine erste Richtung in eine erste Funktionsstellung verschoben wird und beim Verschwenken des Betätigungshebels in eine hierzu entgegengesetzte Richtung in eine zweite Funktionsstellung verschoben wird. Gegenüber einer direkten manuellen Verschiebung des Betätigungselements in seine beiden Funktionsstellungen weist die Verwendung des Betätigungshebels zu diesem Zweck den Vorteil auf, dass das Betätigungselement mit einem erheblich geringeren Kraftaufwand verschoben werden kann, sodass das Verschieben des Betätigungselement in seine Funktionsstellungen deutlich leichter ist.

Zweckmäßigerweise liegt bei der erfindungsgemäßen Handhabe eine Verschiebeachse des Betätigungselements in einer Schwenkebene des Betätigungshebels. D. h. der Schwenkhebel ist derart an der Handhabe angeordnet, dass eine Schwenkbewegung des Betätigungshebels und die Verschiebebewegung des Betätigungselements in einer gemeinsamen Ebene erfolgen. Diese Ausgestaltung hat den Vorteil, dass mit dem Betätigungshebel bei dessen Verschwenken, eine direkt in Verschieberichtung des Betätigungselements wirkende Kraftkomponente auf das Betätigungselement ausgeübt wird.

Die Bewegungskopplung des Betätigungshebels mit dem Betätigungselement erfolgt bei der erfindungsgemäßen Handhabe bevorzugt durch einen auch bei der gemeinsamen Bewegung von Betätigungshebel und Betätigungselement nicht lösbaren Formschluss zwischen dem Betätigungselement und dem Betätigungshebel. In diesem Sinne ist gemäß einer vorteilhaften Weiterbildung der Handhabe vorgesehen, dass das Betätigungselement eine Verzahnung aufweist, in die eine an dem Betätigungshebel ausgebildete korrespondierende Verzahnung eingreift. Aufgrund der linearen Bewegungsführung des Betätigungselements und der schwenkbeweglichen Anordnung des Betätigungshebels um eine Schwenk- bzw. Drehachse, ist die Verzahnungspaarung von Betätigungselement und Betätigungshebel besonders vorteilhaft ähnlich einer Zahnstange-Zahnrad-Paarung ausgebildet. So kann an einer Außenseite des Betätigungselements eine zahnstangenähnliche Verzahnung ausgebildet sein und an einer Außenseite des Betätigungshebels und vorzugsweise am Ende des zweiten Hebelarms eine Verzahnung vorgesehen sein, die der Verzahnung eines Abschnitts eines Zahnrads entspricht.

Gemäß einer weiteren vorteilhaften Ausgestaltung kann das Betätigungselement der erfindungsgemäßen Handhabe zumindest zwei Vertiefungen aufweisen, in die in den zumindest zwei Funktionsstellungen des Betätigungselements ein an der Handhabe feststehend angeordnetes federvorgespanntes Rastelement eingreift. Diese Ausgestaltung dient dazu, das Betätigungselement in seinen Funktionsstellungen gegen eine unbeabsichtigte Linearbewegung zu sichern. Bevorzugt weist das Betätigungselement eine weitere Vertiefung auf, die an dem Betätigungselement derart angeordnet ist, dass das Rastelement in sie in einer Stellung einrastet, in der das Betätigungselement keine Funktion ausführt. Das Rastelement ist an der Handhabe vorzugsweise derart angeordnet, dass ein auf einem Federelement ruhender Rastkörper des Rastelements von dem Federelement in eine Richtung normal zur Verschieberichtung des Betätigungselements gedrückt wird. Die an dem Betätigungselement ausgebildeten Vertiefungen erstrecken sich hierbei an der Außenseite des Betätigungselements zweckmäßigerweise auch normal zur Verschieberichtung des Betätigungselements, wobei sie vorteilhaft so dimensioniert sind, dass das Rastelement in sie formschlüssig eingreift. Die Vertiefungen können gleichzeitig Teil der vorangehend beschriebenen Verzahnung für den Eingriff des Betätigungshebels sein.

Bevorzugt ist der Betätigungshebel an einer Seite der Handhabe angeordnet, an der sich ein feststehend angeordnetes Griffteil der Handhabe im Wesentlichen normal zu einer Hauptachse der Handhabe erstreckt. Hierbei ist unter der Hauptachse der Handhabe eine Achse der Handhabe zu verstehen, die sich in Richtung der Längsausdehnung eines medizinischen Instruments, dessen Teil die Handhabe bildet, erstreckt. D. h. bezogen auf das medizinische Instrument, in dem die erfindungsgemäße Handhabe angeordnet ist, ist das feststehend angeordnete Griffteil in Art eines Pistolengriffs angeordnet, wobei der Betätigungshebel vorzugsweise ähnlich dem Abzugshebel einer Pistole vor dem Griffteil angeordnet ist. Diese Ausgestaltung ermöglicht eine einfach Zugänglichkeit des Betätigungshebels bei einhändiger Bedienung der Handhabe.

Vorteilhafterweise ist das Betätigungselement der Handhabe ein Drehrad, welches in der Handhabe axial verschiebbar angeordnet ist. D. h. das Drehrad ist in seine zumindest zwei Funktionsstellungen in Richtung einer Mittelachse bzw. Drehachse des Drehrads verschiebbar, wobei in den beiden Funktionsstellungen die der jeweiligen Funktionsstellung zugeordnete Funktion durch Drehen des Drehrads ausgeführt werden kann.

Weiter vorteilhaft ist das Drehrad bevorzugt auf einer Hülse angeordnet. Diese Hülse ist in der Handhabe in Richtung ihrer Mittelachse verschiebbar und um ihre Mittelachse drehbar gelagert. An dem Außenumfang der Hülse sind bevorzugt direkt aneinander angrenzend mehrere um den gesamten Umfang der Hülse umlaufende Nuten ausgebildet, die eine Verzahnung bilden. In diese Verzahnung greift die bevorzugt an dem Betätigungshebel ausgebildete korrespondierende Verzahnung ein. Die Anzahl der an der Außenseite der Hülse angeordneten Nuten entspricht vorzugsweise der Anzahl der Funktionsstellungen des von der Hülse und dem daran angeordneten Drehrad gebildeten Betätigungselements. So können an der Hülse beispielsweise drei Nuten ausgebildet sein, wobei in einer ersten Funktionsstellung des Betätigungselements ein erster Zahn der an dem Betätigungshebel ausgebildeten Verzahnung in eine erste Nut eingreift und nach dem Verschwenken des Betätigungshebels, das mit einer Verschiebung der Hülse in eine Stellung einhergeht, in der das Betätigungselement keine Funktion ausführen kann, ein zweiter Zahn der an dem Betätigungshebel ausgebildeten Verzahnung in eine zweite Nut eingreift und schließlich bei einem weiteren Verschwenken des Betätigungshebels, durch das das Betätigungselement in eine zweite Funktionsstellung verschoben wird, ein dritter Zahn der an dem Betätigungselement ausgebildeten Verzahnung in eine dritte Nut der Hülse eingreift.

Die an der Hülse ausgebildeten Nuten weisen vorzugsweise eine abgerundete Querschnittskontur auf. Bevorzugt entspricht das Profil der Nuten hierbei dem Profil eines flachen Kreissegments. Insbesondere in Verbindung mit solchen Nuten wird die Verzahnung an dem Betätigungshebel vorteilhaft von mehreren in Schwenkrichtung des Hebels direkt aneinander angrenzenden abgerundeten Erhebungen gebildet. Hierbei entspricht das Querschnittsprofil der Erhebungen zweckmäßigerweise im Wesentlichen dem Querschnittsprofil der an der Hülse ausgebildeten Nuten. Die abgerundete Ausgestaltung der an der Hülse ausgebildeten Nuten und die hiermit korrespondierende abgerundete Ausgestaltung der an dem Betätigungshebel ausgebildeten Erhebungen ermöglicht ein unproblematisches Abwälzen der an dem Betätigungshebel angeordneten Verzahnung an der an der Hülse angeordneten Verzahnung unter gleichzeitiger Verschiebung der Hülse bzw. des Betätigungselements.

Zweckmäßigerweise dienen die an der Hülse ausgebildeten Nuten auch zum Festlegen des Betätigungselements in seinen Funktionsstellungen. Hierzu ist das bevorzugt vorgesehene federvorgespannte Rastelement vorteilhaft derart an der Handhabe angeordnet, dass es in den einzelnen Funktionsstellungen des Drehrads jeweils in eine der an der Hülse ausgebildeten Nuten eingreift. In der Handhabe ist das Rast-element vorzugsweise an der von dem Betätigungshebel abgewandten Seite der Hülse angeordnet, wobei das Federelement, auf dem sich der Rastkörper des Rastelements abstützt, den Rastkörper in Richtung der Hülse und somit in Richtung der daran ausgebildeten Nuten drückt. Bei einer Ausgestaltung, bei der ein Drehrad das Betätigungselement bildet, kann der Betätigungshebel vorteilhaft an der Handhabe an einem das Drehrad außenseitig umgebenden Bügel angelenkt sein. Der Bügel umgibt das Drehrad zweckmäßigerweise in Richtung der Mittelachse des Drehrads mit einem gewissen Abstand und bildet so einen mechanischen Schutz für das Drehrad. An dem Bügel kann ein Längsschlitz ausgebildet sein, in dem der Betätigungshebel mit Abstand von dem Drehrad und mit Abstand zu der Hülse, auf der das Drehrad angeordnet ist, derart angelenkt ist, dass der zweite Hebelarm des Betätigungshebels, der zum manuellen Bedienen des Betätigungshebels dient, an der von der Hülse abgewandten Seite des Bügels aus diesem herausragt und die an dem ersten Hebelarm des Betätigungshebels ausgebildete Verzahnung mit der an der Hülse ausgebildet Verzahnung in Eingriff ist.

In alternativer Ausgestaltung zu einer Bewegungskopplung von Betätigungshebel und Betätigungselement mittels an dem Betätigungshebel und an dem Betätigungselement ausgebildeten Verzahnungen kann der Betätigungshebel vorteilhaft gabelförmig ausgebildet sein und sich in einem Abschnitt in zwei voneinander beabstandete Schenkel verzweigen, die einen Bereich des Betätigungselements umgreifen und dort mit dem Betätigungselement bewegungsgekoppelt sind. Die Bewegungskopplung des Betätigungselements mit den beiden Schenkeln des Betätigungshebels kann dadurch erfolgen, dass an der Außenseite des Betätigungselements eine sich über den gesamten Umfang des Betätigungselements erstreckende Nut ausgebildet ist, in die jeweils ein an den beiden Schenkeln angeordneter Vorsprung oder Stift eingreift.

Nachfolgend ist die erfindungsgemäße Handhabe anhand eines in den Zeichnungen dargestellten Ausführungsbeispiels näher erläutert. In den Zeichnungen zeigt:
- Fig. 1: eine Handhabe für ein medizinisches Instrument in einer Seitenansicht,
- Fig. 2: ein medizinisches Instrument mit der Handhabe nach Fig. 1 in einer Seitenansicht,
- Fig. 3: das medizinische Instrument nach Fig. 2 in einer Draufsicht,
- Fig. 4: die Handhabe nach Fig. 1 in einer teilgeschnittenen Darstellung,
- Fig. 5: ein Betätigungselement der Handhabe nach Fig. 1 in einer geschnittenen Seitenansicht,
- Fig. 6: eine Einzelheit Y aus Fig. 4,
- Fig. 7: eine Einzelheit Z aus Fig. 4,
- Fig. 8: die Handhabe nach Fig. 1 in einer teilgeschnittenen Darstellung mit einem Betätigungselement in einer ersten Funktionsstellung und
- Fig. 9: die Handhabe nach Fig. 1 in einer teilgeschnittenen Darstellung mit einem Betätigungselement in einer zweiten Funktionsstellung.

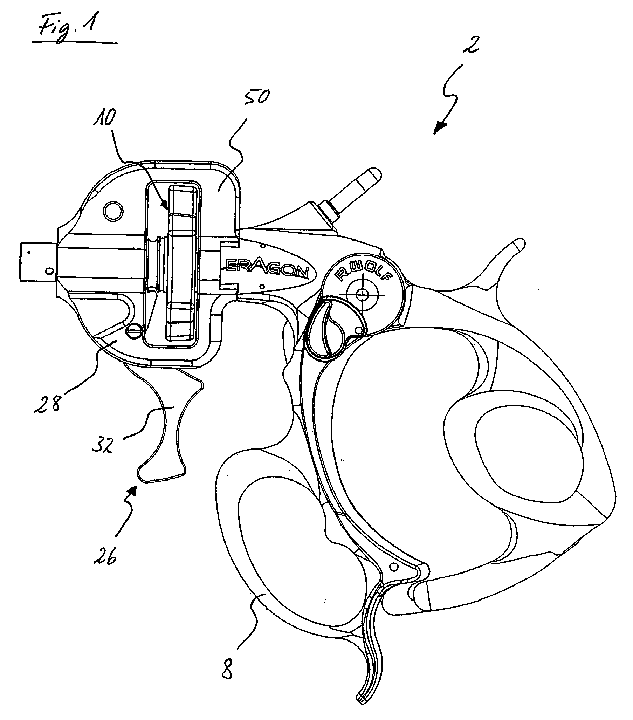

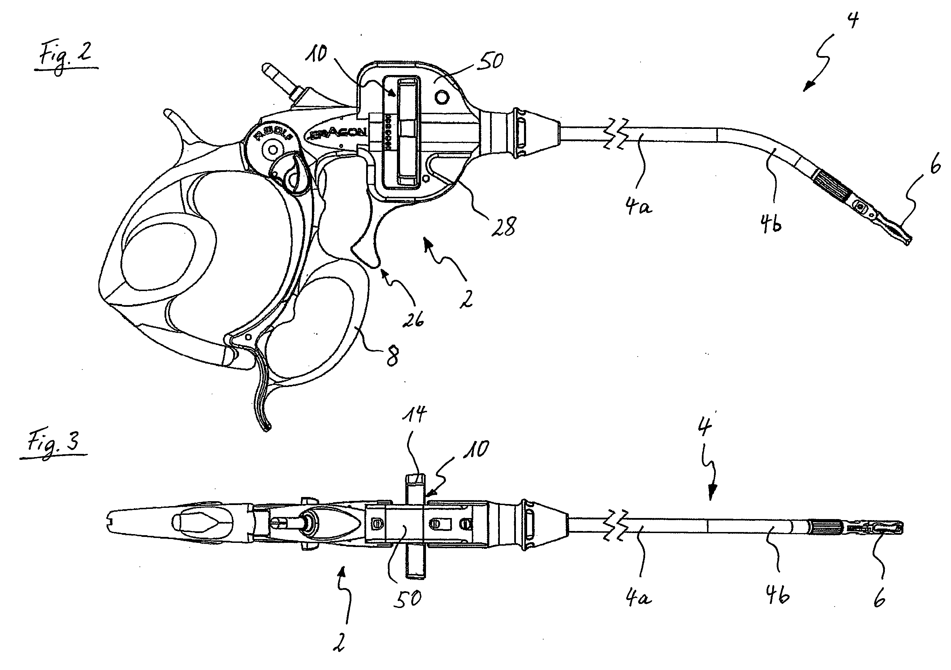

Die in Fig. 1 dargestellte Handhabe 2 ist Teil eines endoskopischen Hohlschaftinstruments, wie es in den Fig. 2 und 3 dargestellt ist. Bei diesem Hohlschaftinstrument ist distalseitig der Handhabe 2 ein starrer Hohlschaft 4 angeordnet, der einen gerade ausgerichteten Hohlschaftabschnitt 4a und einen sich daran anschließenden schräg zum Schaftabschnitt 4a gebogenen distalen Schaftabschnitt 4b aufweist. An dem distalen Ende des Hohlschafts 4 ist ein Werkzeug 6 in Form einer Fasszange angeordnet. Der Hohlschaft 4 ist an der Handhabe 2 um die Längsachse des proximalen Schaftabschnitts 4a drehbar gelagert, also relativ zu der Handhabe 2 drehbar. Das Werkzeug 6 wiederum ist relativ zu dem Hohlschaft 4 drehbar.

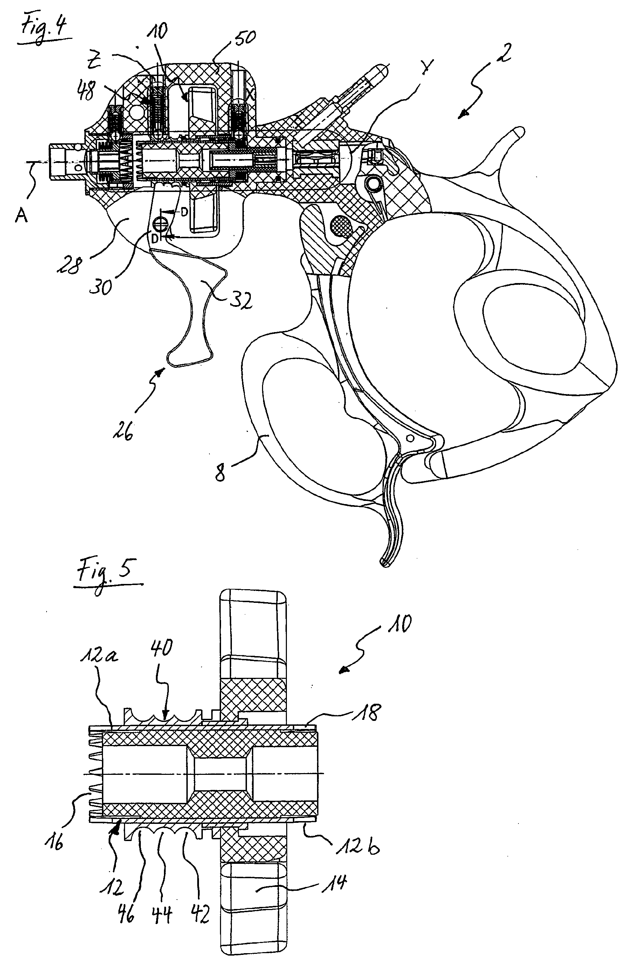

Zum Drehen den Hohlschafts 4 und des Werkzeugs 6 weist die Handhabe 2 ein Betätigungselement 10 auf, das zwischen einem an der Handhabe 2 feststehend angeordneten Griffteil 8, das sich im Wesentlichen normal zu einer Hauptachse A (Fig. 4) der Handhabe 2 erstreckt, und dem distalen Ende der Handhabe 2, an dem der Hohlschaft 4 aus der Handhabe 2 herausgeführt ist, angeordnet ist. Wie insbesondere Fig. 5 zu entnehmen ist, wird das Betätigungselement 10 von einer Hülse 12 und einem darauf angeordneten Drehrad 14 gebildet. In Richtung einer Mittelachse des Betätigungselements 10, deren Lage in Einbaulage des Betätigungselements 10 in der Handhabe 2 mit der Lage der Hauptachse A der Handhabe 2 übereinstimmt, ragen Abschnitte 12a und 12b der Hülse 12 über die Stirnseiten des Drehrads 14 hinaus. An den Stirnseiten der Hülse 12 sind distalseitig eine Verzahnung 16 und proximalseitig eine Verzahnung 18 ausgebildet.

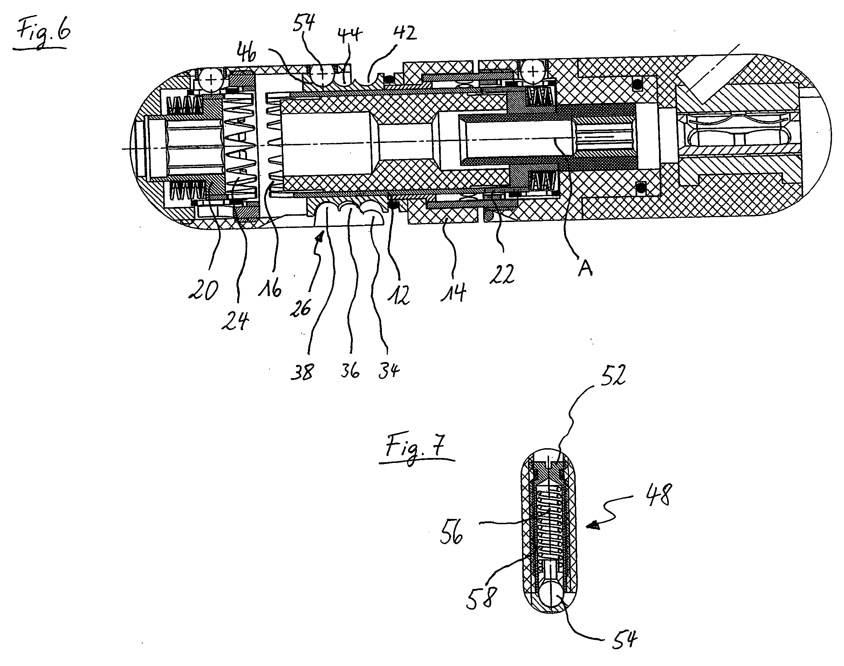

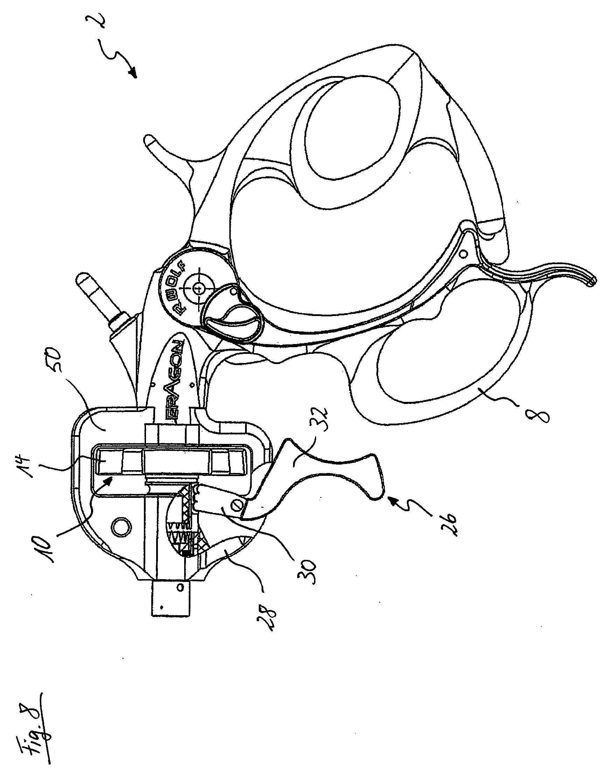

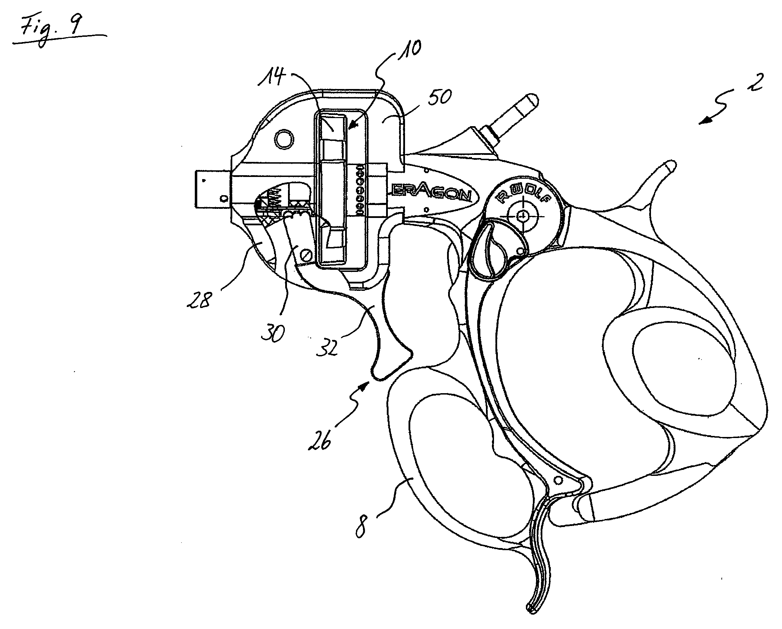

In der Handhabe 2 ist das Betätigungselement 10 um seine Mittelachse, d. h. um die Hauptachse A der Handhabe drehbar gelagert, und in einem begrenzten Bereich in Richtung dieser Mittelachse verschiebbar (siehe Fig. 8 und 9). Wie aus Fig. 6 hervorgeht, wird der axiale Verschiebeweg des Betätigungselements 10 in distaler Richtung durch eine Nabe 20 begrenzt, die mit dem Hohlschaft 4 bewegungsgekoppelt ist. Eine Nabe 22, die mit einer in den Zeichnungen nicht dargestellten Betätigungsstange des Werkzeugs 6 bewegungsgekoppelt ist, begrenzt den Verschiebeweg des Betätigungselements 10 in proximaler Richtung. Sowohl die Nabe 20 als auch die Nabe 22 sind jeweils mit einer Verzahnung ausgestattet. So weist die Nabe 20 an ihrem proximalen Ende eine Verzahnung 24 auf und die Nabe 22 an ihrem distalen Ende eine aus den Zeichnungen nicht ersichtliche Verzahnung auf.

In den Fig. 6 und 8 ist das Betätigungselement 10 in eine proximale End-stellung axial verschoben dargestellt. Hierbei handelt es sich um eine erste Funktionsstellung des Betätigungselements 10, in der das Betäti-gungselement 10 durch Eingriff der proximalseitig der Hülse 12 ausgebildeten Verzahnung 18 in die an der Nabe 22 ausgebildete Verzahnung drehfest mit dem Werkzeug 6 verbunden ist, sodass das Werkzeug 6 durch Drehen des Drehrads 14 relativ zu dem Hohlschaft 4 verdreht werden kann. Wird das Betätigungselement 10 in distale Richtung axial verschoben, greift in einer zweiten Funktionsstellung des Betätigungselements 10 die distalseitig an der Hülse 12 ausgebildete Verzahnung 16 in die Verzahnung 24 der Nabe 20 ein. Hierdurch ist das Betätigungselement 10 mit dem Hohlschaft 4 drehfest verbunden, sodass der Hohlschaft 4 durch Drehen des Drehrads 14 relativ zu der Handhabe 2 verdreht werden kann.

Zum axialen Verschieben des Betätigungselements 10 weist die Handhabe 2 einen Betätigungshebel 26 auf. Dieser Betätigungshebel 26 ist an einem das Drehrad 14 des Betätigungselements 10 umfassenden Bügel 28 schwenkbar neben dem Handgriff 8 gelagert. Der Betätigungshebel 26 ist als ein zweiseitiger Hebel ausgebildet und weist einen ersten Hebelarm 30 und einen zweiten Hebelarm 32 auf, wobei der erste Hebelarm 30 zur nachfolgend eingehender erläuterten Bewegungskopplung des Betätigungshebels 26 mit dem Betätigungselement 10 dient und der zweite Hebelarm 32 den Bedienteil des Betätigungshebels 26 bildet.

An dem von dem zweiten Hebelarm 32 abgewandten Ende des ersten Hebelarms 30 des Betätigungshebels 26 sind drei direkt in Reihe nebeneinander angeordnete Vorsprünge ausgebildet, die von abgerundeten Erhebungen 34, 36 und 38 gebildet werden (siehe Fig. 6). Zusammen bilden die Erhebungen 34, 36 und 38 eine Verzahnung. Die Hülse 12 des Betätigungselements 10 ist in dem Abschnitt 12a distalseitig des Drehrads 14 von einem starr mit der Hülse 12 verbundenen Ring 40 umgeben. An dem Außenumfang des Rings 40 sind in Reihe direkt nebeneinander drei Nuten 42, 44 und 46 ausgebildet, die sich jeweils über den gesamten Außenumfang des Rings 40 erstrecken. Die Nuten 42, 44 und 46 weisen jeweils eine abgerundete Querschnittskontur auf, die mit der Querschnittskontur der an dem ersten Hebelarm 30 des Betätigungshebels 26 ausgebildeten Erhebungen 34, 36 und 38 korrespondiert. Die Nuten 42, 44 und 46 bilden eine Verzahnung, in der die seitens des Betätigungshebels 26 von den Erhebungen 34, 36 und 38 gebildete Verzahnung in Eingriff ist. Dies wird aus den Fig. 4, 6, 8 und 9 deutlich.

In den Fig. 4, 6 und 8 ist der Betätigungshebel 26 in distaler Richtung in eine Endstellung verschwenkt, in der die an dem Hebelarm 30 distal angeordnete Erhebung 38 in die an dem Ring 40 distal angeordnete Nut 36 eingreift. Hierbei ist das Betätigungselement 10 weitestmöglich in proximaler Richtung verschoben, wobei die an der Hülse 12 ausgebildete Verzahnung 18 in die an der Nabe 22 ausgebildete Verzahnung eingreift. Hierdurch ist das Betätigungselement 10 drehfest mit der Nabe 22 und damit einhergehend mit dem Werkzeug 6 drehfest verbunden.

Wird der Betätigungshebel 26 nun in proximale Richtung bzw. in Richtung des Griffteils 8 verschwenkt, kommt die Erhebung 38 außer Eingriff und die Erhebung 36 greift unter Verschiebung des Betätigungselements 10 in distaler Richtung in die an dem Ring 40 ausgebildete mittlere Nut 44 ein. In dieser Stellung ist das Betätigungselement 10 weder mit der Nabe 22 noch mit der Nabe 20 gekoppelt.

Verschwenkt man den Betätigungshebel weiter in Richtung des Griffteils 8 kommt die Erhebung 36 außer Eingriff und die Erhebung 34 greift unter Verschiebung des Betätigungselements 10 in distaler Richtung in die an dem Ring 40 proximalseitig ausgebildete Nut 42 ein. Die an der Hülse 12 des Betätigungselements 10 ausgebildete Verzahnung 16 greift nun in die an der Nabe 20 ausgebildete Verzahnung 24 ein, sodass das Betätigungselement 10 mit der Nabe 20 und somit mit dem Hohlschaft 4 des medizinischen Instruments drehfest verbunden ist (Fig. 9).

Wie beschrieben, ist das Betätigungselement 10 mit dem Betätigungshebel 26 aufgrund der drei an dem Hebelarm 30 des Betätigungshebels 26 ausgebildeten drei Erhebungen 34, 36 und 38 und der drei an dem Ring 40 der Hülse 12 ausgebildeten Nuten 42, 44 und 46 in drei Funktionsstellungen verschiebbar, nämlich in eine erste proximale Funktionsstellung, in der das Betätigungselement 10 drehfest mit dem Werkzeug 6 gekoppelt ist, in eine zweite Funktionsstellung, in der das Betätigungselement weder mit dem Werkzeug noch mit dem Hohlschaft 4 gekoppelt ist und in eine dritte distale Funktionsstellung, in der das Betätigungselement 10 mit dem Hohlschaft 4 drehfest gekoppelt ist.

Um das Betätigungselement 10 in allen drei Funktionsstellungen fixieren zu können, weist die Handhabe 2 ein Rastelement 48 auf (Fig. 7). Das Rastelement 48 ist an einem das Drehrad 14 des Betätigungselements 10 umgebenden Bügel 50 angeordnet, der an der Handhabe 2 dem Bügel 28 direkt gegenüberliegend angeordnet ist. Das Rastelement 48 besteht im Wesentlichen aus einer Schraube 52 mit einem Hohlschaft, in dem sich ein Rastkörper 54 auf einer als Druckfeder ausgebildeten Schraubenfeder 56 abstützt.

Das Rastelement 48 ist in einer an dem Bügel 50 ausgebildeten und zu dem Betätigungselement 10 hin offene Gewindebohrung 58 verschraubt, die sich normal zu der Hauptachse A der Handhabe 2 radial in Richtung des Abschnitts 12a der Hülse 12 erstreckt. In den drei Funktionsstellungen des Betätigungselements 10 rastet der Rastkörper 54 des Rastelements 48 diametral gegenüber dem Betätigungshebel 26 jeweils in eine der an dem Ring 40 der Hülse 12 ausgebildeten Nuten 42, 44 bzw. 46 ein.

### Bezugszeichenliste

- 2: - Handhabe
- 4: - Schaft
- 4a, 4b: - Schaftabschnitt
- 6: - Werkzeug
- 8: - Griffteil
- 10: - Betätigungselement
- 12: - Hülse
- 12a, 12b: - Abschnitt
- 14: - Drehrad
- 16: - Verzahnung
- 18: - Verzahnung
- 20: - Nabe
- 22: - Nabe
- 24: - Verzahnung
- 26: - Betätigungshebel
- 28: - Bügel
- 30: - Hebelarm
- 32: - Hebelarm
- 34: - Erhebung
- 36: - Erhebung
- 38: - Erhebung
- 40: - Ring
- 42: - Nut
- 44: - Nut
- 46: - Nut
- 48: - Rastelement
- 50: - Bügel
- 52: - Schraube
- 54: - Rastkörper
- 56: - Schraubenfeder
- A: - Hauptachse
- Y: - Einzelheit
- Z: - Einzelheit

## Patentansprüche

1. Handhabe (2) für ein medizinisches Instrument mit einem an der Handhabe (2) angeordneten Betätigungselement (10), welches zumindest in eine erste und eine zweite Funktionsstellung verschiebbar ist und sowohl in der ersten als auch in der zweiten Funktionsstellung zur Ausübung einer Funktion in einer Ebene quer zu seiner Verschieberichtung bewegbar ist, **dadurch gekennzeichnet, dass** an der Handhabe (2) ein schwenkbarer Betätigungshebel (26) angeordnet ist, welcher zum Verschieben des Betätigungselements (10) in die zumindest zwei Funktionsstellungen mit dem Betätigungselement (10) bewegungsgekoppelt ist.

2. Handhabe (2) nach Anspruch 1 **dadurch gekennzeichnet, dass** eine Verschiebeachse des Betätigungselements (10) in einer Schwenkebene des Betätigungshebels (26) liegt.

3. Handhabe (2) nach einem der vorangehenden Ansprüche, **dadurch gekennzeichnet, dass** das Betätigungselement (10) eine Verzahnung aufweist, in welche eine an dem Betätigungshebel (26) ausgebildete Verzahnung eingreift.

4. Handhabe (2) nach einem der vorangehenden Ansprüche, **dadurch gekennzeichnet, dass** das Betätigungselement (10) zumindest zwei Vertiefungen aufweist, in die in den zumindest zwei Funktionsstellungen des Betätigungselements (10) ein an der Handhabe (2) feststehend angeordnetes, federvorgespanntes Rastelement (48) eingreift.

5. Handhabe (2) nach einem der vorangehenden Ansprüche, **dadurch gekennzeichnet, dass** der Betätigungshebel (26) an einer Seite der Handhabe (2) angeordnet ist, an der sich ein feststehend angeordnetes Griffteil (8) der Handhabe (2) im Wesentlichen normal zu einer Hauptachse (A) der Handhabe (2) erstreckt.

6. Handhabe (2) nach einem der vorangehenden Ansprüche, **dadurch gekennzeichnet, dass** das Betätigungselement (10) ein Drehrad (14) ist, welches in der Handhabe (2) axial verschiebbar angeordnet ist.

7. Handhabe (2) nach Anspruch 6, **dadurch gekennzeichnet, dass** das Drehrad (14) auf einer Hülse (12) angeordnet ist, wobei an dem Außenumfang der Hülse (12) direkt aneinander angrenzend mehrere um den gesamten Umfang der Hülse umlaufende Nuten (42, 44, 46) ausgebildet sind, welche eine Verzahnung bilden.

8. Handhabe (2) nach Anspruch 7, **dadurch gekennzeichnet, dass** die Nuten (42, 44, 46) eine abgerundete Querschnittskontur aufweisen.

9. Handhabe (2) nach einem der Ansprüche 4 bis 8, **dadurch gekennzeichnet, dass** die Verzahnung an dem Betätigungshebel (26) von mehreren in Schwenkrichtung des Betätigungshebels (26) direkt aneinander angrenzenden abgerundeten Erhebungen (34, 36, 38) gebildet wird.

10. Handhabe (2) nach einem der Ansprüche 7 bis 9, **dadurch gekennzeichnet, dass** das federvorgespannte Rastelement (48) derart an der Handhabe (2) angeordnet ist, dass es in den einzelnen Funktionsstellungen des Drehrads (14) jeweils in eine an der Hülse (12) ausgebildete Nut (42, 44, 46) eingreift.

11. Handhabe (2) nach einem der Ansprüche 6 bis 10, **dadurch gekennzeichnet, dass** der Betätigungshebel (26) an einem das Drehrad (14) außenseitig umgebenden Bügel (28) angelenkt ist.

12. Handhabe (2) nach einem der Ansprüche 1, 2 sowie 4 bis 6, **dadurch gekennzeichnet, dass** der Betätigungshebel (26) gabelförmig ausgebildet ist und in einem Abschnitt in zwei voneinander beabstandete Schenkel verzweigt, welche einen Bereich des Betätigungselements (10) umgreifen und dort mit dem Betätigungselement (10) bewegungsgekoppelt sind.

## Claims

1. A handle (2) for a medical instrument with an actuation element (10) which is arranged on the handle (2) and which is displaceable into at least a first and a second functional position and can be moved in a plane transverse to its displacement direction, in the first as well as second functional position for carrying out a function, **characterised in that** a pivotable actuation lever (26) is arranged on the handle (2), said actuation lever being coupled in movement to the actuation element (10) for displacing the actuation element (10) into the at least two functional positions.

2. A handle (2) according to claim 1, **characterised in that** a displacement axis of the actuation element (10) lies in a pivot plane of the actuation lever (26).

3. A handle (2) according to one of the preceding claims, **characterised in that** the actuation element (10) comprises a toothing, into which a toothing formed on the actuation lever (26) engages.

4. A handle (2) according to one of the preceding claims, **characterised in that** the actuation element (10) comprises at least two recesses, into which a spring-biased locking element (48) fixedly arranged on the handle (2) engages, in the at least two functional positions of the actuation element (10).

5. A handle (2) according to one of the preceding claims, **characterised in that** the actuation lever (26) is arranged at a side of the handle (2), at which a fixedly arranged grip part (8) of the handle (2) extends essentially normally to a main axis (A) of the handle (2).

6. A handle (2) according to one of the preceding claims, **characterised in that** the actuation element (10) is a rotation wheel (14) which is arranged in an axially displaceable manner in the handle (2).

7. A handle (2) according to claim 6, **characterised in that** the rotation wheel (14) is arranged on a sleeve (12), wherein several grooves (42, 44, 46) which are peripheral around the whole periphery of the sleeve and which form a toothing are formed directly adjacently one another on the outer periphery of the sleeve (12).

8. A handle (2) according to claim 7, **characterised in that** the grooves (42, 44, 46) have a rounded cross-sectional contour.

9. A handle (2) according to one of the claims 4 to 8, **characterised in that** the toothing on the actuation lever (26) is formed by several rounded prominences (34, 36, 38) which are directly adjacent one another in the pivot direction of the actuation lever (26).

10. A handle (2) according to one of the claims 7 to 9, **characterised in that** the spring-biased locking element (48) is arranged on the handle (2) in a manner such that it engages in each case into a groove (42, 44, 46) formed on the sleeve (12) in the individual functional positions of the rotation wheel (14).

11. A handle (2) according to one of the claims 6 to 10, **characterised in that** the actuation lever (26) is articulated on a bow (28) surrounding the rotation wheel (14) on the outside.

12. A handle (2) according to one of the claims 1, 2 as well as 4 to 6, **characterised in that** the actuation lever (26) is designed in a fork-like manner and in a section branches into two limbs which are distanced to one another, encompass a region of the actuation element (10) and are coupled in movement there to the actuation element (10).

## Revendications

1. Poignée (2) pour un instrument médical avec un élément d'actionnement (10) sur la poignée (2) qui est apte à être coulissé pour le moins dans une première et une deuxième positions de fonctionnement et qui est déplaçable, aussi bien dans la première que dans la deuxième positions de fonctionnement, pour mettre en oeuvre une fonction, dans un plan transversal à sa direction de coulissement, **caractérisée en ce qu'**un levier d'actionnement pivotant (26) est disposé sur la poignée d'actionnement (2) qui est relié en mouvement à l'élément d'actionnement (10) pour le coulissement de l'élément d'actionnement (10) dans lesdites au moins deux positions de fonctionnement.

2. Poignée (2) selon la revendication 1, **caractérisée en ce qu'**un axe de coulissement de l'élément d'actionnement (10) est situé dans un plan de pivotement du levier d'actionnement (26).

3. Poignée (2) selon une des revendications précédentes, **caractérisée en ce que** l'élément d'actionnement (10) comprend une indentation dans laquelle s'engage une indentation formée sur le levier d'actionnement (26).

4. Poignée (2) selon l'une des revendications précédentes, **caractérisée en ce que** l'élément d'actionnement (10) comprend au moins deux enfoncements dans lesquels s'engage, dans lesdites au moins deux positions de fonctionnement de l'élément d'actionnement (10), un élément d'encliquetage (48) précontraint par ressort et disposé de manière fixe sur la poignée (2).

5. Poignée (2) selon l'une des revendications précédentes, **caractérisée en ce que** le levier d'actionnement (26) est disposé sur un côté de la poignée (2) sur lequel s'étend une partie de saisie (8) disposée de manière fixe de la poignée (2), sensiblement perpendiculairement par rapport à un axe principal (A) de la poignée (2).

6. Poignée (2) selon l'une des revendications précédentes, **caractérisée en ce que** l'élément d'actionnement (10) est une roue tournante (14) qui est disposée de manière axialement coulissante dans la poignée (2).

7. Poignée (2) selon la revendication 6, **caractérisée en ce que** la roue tournante (14) est disposée sur une douille (12), plusieurs rainures (42, 44, 46) circonférentielles sur le pourtour complet de la douille et adjacentes les unes aux autres, qui forment une indentation, étant formées sur le pourtour extérieur de la douille (12).

8. Poignée (2) selon la revendication 7, **caractérisée en ce que** les rainures (42, 44, 46) présentent un contour de section transversale arrondi.

9. Poignée (2) selon l'une des revendications 4 à 8, **caractérisée en ce que** l'indentation sur le levier d'actionnement (26) est formée par plusieurs proéminences arrondies (34, 36, 38) directement adjacentes dans la direction de pivotement du levier d'actionnement (26).

10. Poignée (2) selon l'une des revendications 7 à 9, **caractérisée en ce que** l'élément d'encliquetage (48) précontraint par ressort est disposé sur la poignée (2) de manière telle qu'il s'engage dans les différentes positions de fonctionnement de la roue tournante (14) à chaque fois dans une rainure (42, 44, 46) formée dans la douille (12).

11. Poignée (2) selon l'une des revendications 6 à 10, **caractérisée en ce que** le levier d'actionnement (26) est articulé sur un arceau (28) entourant la roue tournante (14) du côté extérieur.

12. Poignée (2) selon l'une des revendications 1, 2 et 4 à 6, **caractérisée en ce que** le levier d'actionnement (26) est formé selon une forme fourchue et se divise dans une partie en deux branches espacées l'une de l'autre qui entourent une zone de l'élément d'actionnement (10) et y sont accouplées en mouvement à l'élément d'actionnement (10).
